# EUROPEAN PATENT APPLICATION

(11) **EP 4 151 724 A1**
(43) Date of publication of application: **22.03.2023**
(21) Application number: 21805293.4
(22) Date of filing: 10.05.2021
(51) Int. Cl.: C12N 5/0793, C12N 15/63, A61P 25/00

(54) **METHOD FOR INDUCING GLIAL CELLS TRANSDIFFERENTIATION INTO FUNCTIONAL NEURONS, AND APPLICATION THEREOF**

(30) Priority: 12.05.2020 CN 202010399397
(71) Applicant: Neuragen Biotherapeutics (Suzhou) Co., Ltd., Suzhou, Jiangsu 215127 (CN)
(72) Inventor: CHEN, Rulei, Shanghai 201203 (CN); LIU, Ting, Shanghai 201203 (CN)
(74) Representative: Engelhard, Markus
(86) International application number: PCT/CN2021/092851
(87) International publication number: WO 2021/228050

(57) **Abstract**

Provided is a use of a Neurog2 functional fragment. The functional fragment can induce, in vivo or in vitro, glial cell formation into functional neuron cells, and thus can not only have a transdifferentiation function in normal tissue, but also facilitate neural reconstruction of damaged neural tissue.

## Description

### TECHNICAL FIELD

The present invention belongs to the fields of biotechnology and gene therapy, in particular, the present invention relates to a method and application for inducing the transdifferentiation of glial cells into functional neuron cells, especially the application in the repair of spinal cord injury.

### BACKGROUND OF THE INVENTION

The main pathological changes caused by central nervous system injury and various neurodegenerative diseases in mammals are irreversible neuronal degeneration and necrosis and neural circuit damage. How to replace dead and lost neurons in the damaged and diseased brain or spinal cord and rebuild neural circuits is a key step in treatment. Because the central nervous system (brain and spinal cord) of adult mammals has a very limited ability to repair itself, it is difficult to make up for the loss of neuronal cells on its own.

The transplantation of exogenous neurons or neurogenic cells is inefficient, and has potential risks of tumorigenicity and immunogenicity. In recent years, the appearance of cell reprogramming technology has brought revolutionary changes to regenerative medicine. In vivo reprogramming of astrocytes to induce neurons by expressing single or combined multiple neurogenic transcription factors is expected to be an important new strategy for neuron replacement therapy. Therefore, it is very important for brain and spinal cord functional repair to find appropriate transcription factors to induce astrocytes to transdifferentiate into active neurons in vivo. Especially at present, there is no effective method to reverse spinal cord injury.

### SUMMARY OF THE INVENTION

The present invention provides a method for inducing the transdifferentiation of glial cells into functional neuron cells in vitro or in vivo by the functional fragment of Neurog2, and the application thereof in the preparation of a nerve repair pharmaceutical composition.

The first aspect of the present invention provides a Neurog2 functional fragment for use in (i) a preparation of a pharmaceutical composition for nerve induced a glial cell to form a functional neuron; and/or (ii) a preparation of a pharmaceutical composition for a nervous system disease.

In another preferred embodiment, the glial cell is selected from human or non-human mammals.

In another preferred embodiment, the glial cell is selected from the group consisting of astrocyte, NG2 glial cell, oligodendrocyte, microglia or a combination thereof.

In another preferred embodiment, the glial cell is astrocyte.

In another preferred embodiment, the astrocyte includes astrocyte in a normal state and a damaged state.

In another preferred embodiment, the damaged state is neuronal death or apoptosis caused by mechanical trauma, stroke, neurodegenerative disease or other nervous system diseases, thereby blocking or disturbing nerve signal transduction.

In another preferred embodiment, the astrocyte is derived from the spinal cord, the dorsal midbrain or the cerebral cortex, preferably, the astrocyte is derived from the spinal cord and the dorsal midbrain.

In another preferred embodiment, the functional neuron is a cell with the following characteristics: (a) with neuronal morphology, (b) with one or more neuronal markers, and (c) capable of releasing actions potentials and form synaptic connections.

In another preferred embodiment, the functional neuron may also be a group of neurons, specifically, the group of neurons have one or more of the following characteristics:
(a) at least 50% of the neurons, preferably at least 60%, 70%, 80%, 90%, or 100% of the neurons express the neuron marker NeuN;
(b) capable of releasing action potentials and capable of forming synaptic connections.

In another preferred embodiment, the functional neuron is excitatory neuron, preferably VGLUT2⁺ excitatory neuron,

In another preferred embodiment, the functional neuron includes glutamatergic neurons or a group of glutamatergic neurons.

In another preferred embodiment, the functional neuron is capable of releasing action potentials and forming synaptic connections.

In another preferred embodiment, the Neurog2 functional fragment is a functional Neurog2 protein or a nucleic acid sequence encoding a Neurog2 functional protein;

In another preferred embodiment, the Neurog2 functional fragment is a protein or nucleic acid sequence that can normally perform the physiological function of the Neurogenin 2 transcription factor.

In another preferred embodiment, Neurog2 is derived from mammals, preferably, from human or non-human primate mammals.

In another preferred embodiment, the GenBank number of the Neurog2 functional fragment is 11924, and the protein sequence is shown in SEQ ID No.: 1; the NCBI Reference Sequence number of the mRNA encoding the Neurog2 gene is NM_ 009718.3, CDS sequence is shown in SEQ ID NO.: 2.

In another preferred embodiment, the GenBank number of the Neurog2 functional fragment is 63973, and the protein sequence is shown in SEQ ID NO.: 3; the NCBI Reference Sequence number of the mRNA encoding the Neurog2 gene is NM_ 024019.4, CDS sequence is shown in SEQ ID No.: 4.

In another preferred embodiment, the sequence homology between the Neurog2 functional fragment and SEQ ID No.: 1 is not less than 83%; more preferably, the sequence homology between the Neurog2 functional fragment sequence and SEQ ID No.: 1 is not less than 90%; preferably, the sequence homology between the Neurog2 functional fragment sequence and SEQ ID NO.: 1 is not less than 95%.

In another preferred embodiment, the nucleotide functional sequence encoding Neurog2 has no less than 80% homology with SEQ ID NO.: 2 sequence; more preferably, the nucleotide functional sequence encoding Neurog2 has no less than 90% homology with SEQ ID NO.: 2 sequence; preferably, the nucleotide functional sequence encoding Neurog2 has no less than 95% homology with SEQ ID NO.: 2.

In another preferred embodiment, the sequence homology between the Neurog2 functional fragment and SEQ ID No.: 3 is not less than 83%; more preferably, the sequence homology between the Neurog2 functional fragment sequence and SEQ ID No.: 3 is not less than 90%; preferably, the sequence homology between the Neurog2 functional fragment sequence and SEQ ID No.: 3 is not less than 95%.

In another preferred embodiment, the nucleotide functional sequence encoding Neurog2 has no less than 80% homology with SEQ ID NO.: 4; more preferably, the nucleotide functional sequence encoding Neurog2 has no less than 90% homology with SEQ ID NO.: 4 sequence; preferably, the nucleotide functional sequence encoding Neurog2 has no less than 95% homology with SEQ ID NO.: 4.

The second aspect of the present invention provides a delivery system containing a Neurog2 functional fragment,

The delivery system can be applied in vitro or in vivo to induce the transdifferentiation of glial cell into functional neuron; the glial cell is derived from spinal cord, dorsal midbrain or cerebral cortex, preferably, the glial cell is derived from the spinal cord and dorsal midbrain; the glial cell is in the normal state or the damaged state.

In another preferred embodiment, the functional fragment of Neurog2 can be passively absorbed by a glial cell or reach the interior of a glial cell through a delivery system to take effect.

In another preferred embodiment, the delivery system contains Neurog2 functional fragments, including but not limited to expression vectors containing Neurog2 functional fragments, nanoparticles containing Neurog2 functional fragments, exosomes containing Neurog2 functional fragments, modified red blood cells or bacteria containing Neurog2 functional fragments targeted effectors with Neurog2 functional fragments (such as glial cell specific antibody, polypeptide or other targeted substances).

In a preferred embodiment, the delivery system is an expression vector carrying a Neurog2 functional fragment, and the expression vector can enter glial cells and express exogenous Neurog2 protein in astrocytes.

In another preferred embodiment, the expression vector includes plasmid and viral vector.

In another preferred embodiment, the expression vector is a viral vector, including but not limited to adenovirus vector, adeno-associated virus vector (AAV), retrovirus expression vector or lentivirus vector, etc., preferably an adeno-associated virus vector (AAV).

In another preferred embodiment, the expression vector is an astrocyte-specific expression vector.

In another preferred embodiment, the expression vector containing the Neurog2 functional fragment also contains a glial cell-specific promoter, and the promoter includes but is not limited to GFAP promoter, NG2 promoter, Aldh1L1 promoter, IBA1 promoter, CNP promoter, LCN2 promoter or genetically engineered promoter variants.

In another preferred embodiment, the expression vector containing the Neurog2 functional fragment also contains one or more regulatory elements for regulating gene expression, for enhancing the expression level of the gene, including but not limited to CMV enhancers, SV40 enhancer, EN1 enhancer or genetically engineered enhancer variants, as well as SV40 polyA tailing signal, human insulin gene polyA tailing signal or WPRE (woodchuck hepatitis B virus post-transcriptional regulatory element), human-derived MAR sequences or genetically engineered variants.

In another preferred embodiment, the expression vector containing the Neurog2 functional fragment can also contains other functional fragments, and the other functional fragments can be reporter genes or other transcription factors with reprogramming function Functional fragments, including but not limited to Ascl1, NeuroD1, etc.

In another preferred embodiment, the expression vector containing the Neurog2 functional fragment is a GFAP-AAV vector; the GFAP-AAV vector carries a viral ITR sequence, a CMV enhancer, a human GFAP promoter, a coding frame of Neurog2 functional fragment and a post-transcriptional regulatory element WPRE, etc.; the expression vector can also contain a reporter gene, but the reporter gene is not necessary in practical applications, as the described GFAP-AAV expression vector is from 5' to 3' end may include the following elements in sequence: viral ITR sequence + enhancer of CMV + promoter of human GFAP + coding frame of Neurog2 and red fluorescent protein mCherry + post-transcriptional regulatory element WPRE + viral ITR sequence + promoter and coding frame of ampicillin resistance gene, wherein the coding frame of the red fluorescent protein mCheny and the promoter and the coding frame of the ampicillin resistance gene are not necessary.

In another preferred embodiment, the expression vector containing the Neurog2 functional fragment is an NG2-lentiviral vector, the NG2-lentiviral vector contains the viral ITR sequence, the promoter of human NG2, and the expression vector of the Neurog2 functional fragment coding frame and post-transcriptional regulatory element WPRE, etc.; the expression vector can also carry a reporter gene, but the reporter gene is not necessary in practical applications, such as the NG2-lentiviral vector from the 5' to 3' end can be the following elements are included in sequence: viral ITR sequence + promoter of human NG2 + coding frame of Neurog2 and green fluorescent protein GFP + post-transcriptional regulatory element WPRE + viral ITR sequence + promoter and coding frame of ampicillin resistance gene, wherein the coding frame of green fluorescent protein GFP and the promoter and the coding frame of ampicillin resistance gene are not necessary.

The third aspect of the present invention provides a host cell comprising an exogenous Neurog2 functional fragment

In another preferred embodiment, the polynucleotide encoding the Neurog2 protein is integrated into the chromosome of the host cell, or the host cell contains the expression vector described in the second aspect of the present invention.

In another preferred embodiment, the host cell is derived from a glial cell, and the delivery system described in the second aspect of the present invention can be used to integrate a polynucleotide encoding Neurog2 protein into its chromosome, or the host cell can be the functional fragment of Neurog2 transferred into the cell promotes the transdifferentiation of the host cell into functional neurons.

In another preferred embodiment, the host cell is derived from the glial cell cultured in vitro.

In a preferred embodiment, the host cell is derived from glial cells under normal and damaged conditions in vivo.

In another preferred embodiment, the glial cell is astrocyte.

In a preferred embodiment, the host cell is a functional neuron or a group of functional neuron cells.

The fourth aspect of the present invention provides a pharmaceutical composition comprising (A) a Neurog2 functional fragment described in the first aspect of the present invention; and/or (B) the delivery system described in the second aspect of the invention, or (C) the host cell described in the third aspect of the present invention; and (D) a pharmaceutically acceptable excipient

The fifth aspect of the present invention provides the use of the delivery system described in the second aspect of the present invention, the host cell described in the third aspect of the present invention, and/or the pharmaceutical composition described in the fourth aspect of the present invention, which can be used for preparation drugs for nervous system damage and nerve repair.

In another preferred embodiment, the nervous system injury includes spinal cord injury, epilepsy, Alzheimer's disease (AD), Parkinson's disease (PD), neuronal death or irreversible loss caused by stroke, etc.

In another preferred embodiment, the nerve repair is achieved by the use of the pharmaceutical composition described in the fourth aspect of the present invention to restore the function of neurons in the damaged area of the nervous system.

A sixth aspect of the present invention provides an in vitro non-therapeutic method for transdifferentiating astrocytes into functional neuron cells, comprising the steps of:

In the presence of exogenous Neurog2 functional fragments, astrocytes are cultured, thereby inducing astrocytes to form functional neuronal cells.

A seventh aspect of the present invention provides a functional neuron cell and/or neuron cell group transdifferentiated from astrocytes, the functional neuron cells and/or neuron cell groups are prepared by the method described in the sixth aspect of the present invention, and the functional neuron cells and/or neuron cell groups have one or more of the following features:
(a) at least 50% of the neurons, preferably at least 60%, 70%, 80%, 90%, or 100% of the neurons express NeuN, a marker of mature neurons;
(b) capable of releasing action potentials and capable of forming synaptic connections.

An eighth aspect of the present invention provides a method for treating nervous system diseases, comprising the steps of:
A safe and effective amount of the pharmaceutical composition described in the fourth aspect of the present invention is administered to a subject in need, thereby treating nervous system diseases.

In another preferred embodiment, the neurological diseases include spinal cord injury, epilepsy, Alzheimer's disease (AD), Parkinson's disease (PD), neuronal death or irreversible loss caused by stroke and the like.

In another preferred embodiment, the subject in need is a human.

In another preferred embodiment, the subject in need suffers from a nervous system disease.

A ninth aspect of the present invention provides a method for (a) screening candidate compounds for the treatment of neurological diseases; and/or (b) screening for candidate compounds inducing the transdifferentiation of astrocytes into functional neuronal cells, wherein It is characterized in that it includes the steps:
(i) adding the test compound to the cell culture system as the test group, and using the cell culture system without the test compound as the control group;
(ii) comparing the expression and/or activity E1 of the Neurog2 gene or its protein in the test group with the expression and/or activity E0 in the control group;

Wherein, when E1 in the test group is significantly higher than E0, it indicates that the tested compound is (a) a candidate compound for the treatment of neurological diseases; and/or (b) a candidate compound for induces the transdifferentiation of astrocytes into functional neuron cells.

In another preferred embodiment, the cells are astrocytes.

In another preferred example, the said significantly higher means that E1 is higher than E0, and there is a statistical difference; preferably, E1≥2E0.

In another preferred embodiment, the method further comprises the steps:
(iii) adding the test compound to the astrocyte culture system as the test group, and using the astrocyte culture system without the test compound as the control group;
(iv) comparing the proportion of astrocytes transformed into functional neurons in the test group to determine whether the test compound is (a) a candidate compound for the treatment of neurological diseases; and/or (b) a candidate compound for screening for inducing astrocytes for the transdifferentiation of glial cells into functional neuronal cells;

Wherein, when the ratio T1 of astrocytes transformed into functional neurons in the test group is significantly higher than the ratio T0 in the control group, it indicates that the test compound is (a) a candidate compound for the treatment of neurological diseases; and/or (b) a candidate compound for screening for inducing transdifferentiation of astrocytes into functional neuronal cells.

In another preferred embodiment, the neurological diseases include spinal cord injury, epilepsy, Alzheimer's disease (AD), Parkinson's disease (PD), neuronal death or irreversible loss caused by stroke and the like.

It should be understood that within the scope of the present invention, the above-mentioned technical features of the present invention and the technical features specifically described in the following (eg, the embodiments) can be combined with each other to form new or preferred technical solutions. Due to space limitations, it is not repeated here.

The above description is only an overview of the technical solution of the present invention, in order to be able to understand the technical means of the present invention more clearly, it can be implemented according to the content of the description, and in order to make the above and other objects, features and advantages of the present invention more obvious and easy to understand, the following specific preferred embodiments, and in conjunction with the accompanying drawings, are described in detail as follows.

### BRIEF DESCRIPTION OF THE DRAWINGS

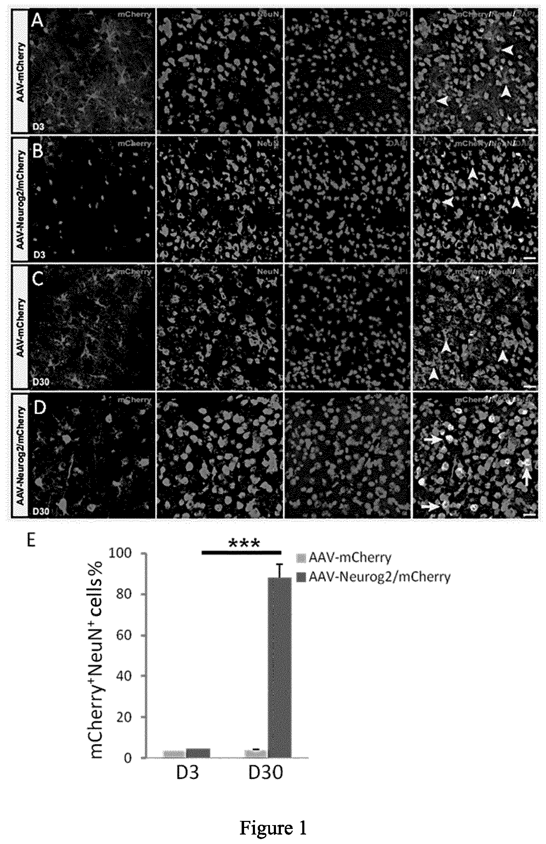

FIG. 1 Neurog2 single factor can efficiently induce midbrain astrocytes into neurons.
Figure 1A, B show that 3 days after injection of control virus AAV-mCherry (negative control) and virus AAV-Neurog2/mCherry into the dorsal midbrain of adult mice, immunoco-labeling showed that neither mCherry co-localized with NeuN.
Figure 1C shows that mCherry still does not co-localize with NeuN 30 days after injection of the control virus AAV-mCherry into the dorsal midbrain of adult mice.
Figure 1D shows that the vast majority of mCherry colocalized with NeuN 30 days after viral AAV-Neurog2/mCherry injection into the dorsal midbrain of adult mice.
Figure 1E shows the statistics of neuron ratios at different times. Arrows and arrowheads represent mCherry⁺NeuN⁺ and mCherry⁺NeuN⁻ cells, respectively. "***" represents p<0.001. Ruler: 50um.

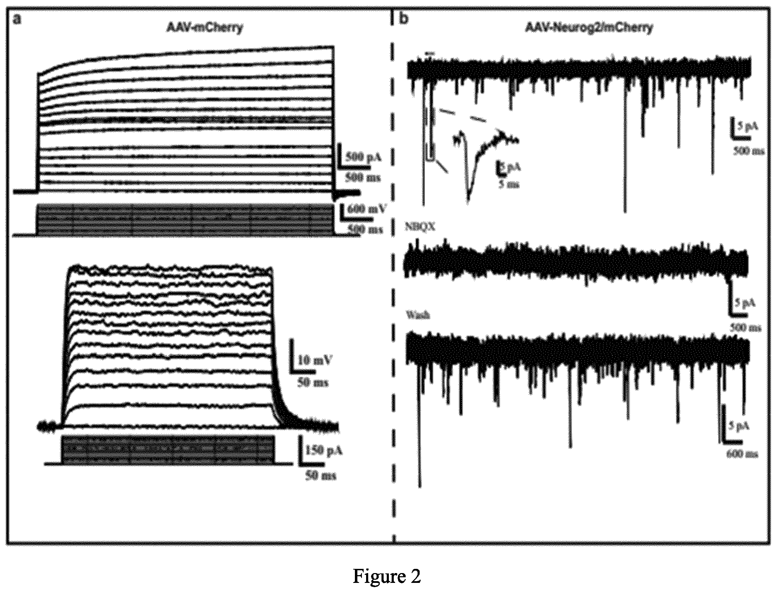

Figure 2 Neurog2-induced neurons in the midbrain were confirmed to be active functional neurons by electrophysiological recordings.Figure 2A shows mCherry⁺ electrophysiological membrane parameters recorded in slices of midbrain 30 days after AAV-mCherry virus infection, membrane current changes recorded in current-clamp mode with gradient voltage stimulation (top) and gradient current stimulation in voltage-clamp mode, respectively Membrane voltage changes were recorded (below). Figure 2B shows the postsynaptic current signal of mCherry⁺ cells recorded 30 days after midbrain infection with AAV-Neurog2/mCherry virus. The postsynaptic current signal disappeared after adding the blocker NBQX, and the postsynaptic current signal after washing out appear again.

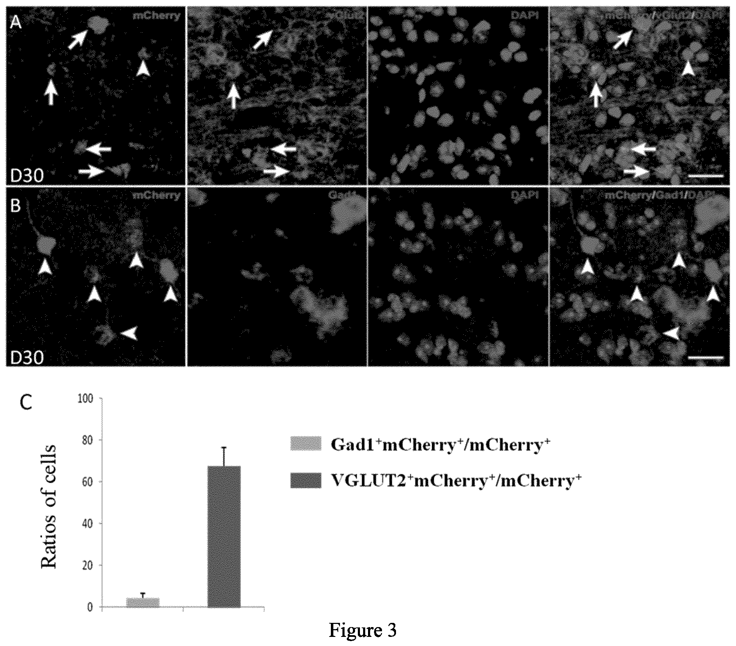

Figure 3 shows Neurog2 midbrain reprogrammed neurons to glutamatergic neurons. Figure 3A, B show the results of in situ histochemical double-labeling of adult mice 30 days after AAV-Neurog2/mCherry virus infection. The overlapping signals of viral signal mCherry protein (red) and VGLUT2 mRNA (A, green) and Gad1 mRNA (B, green) are (right of A) and (right of B), respectively. Arrows in (A) indicate mCherry⁺VGLUT2⁺ double positive cells. Arrows in (B) indicate mCherry⁺Gad1⁻ cells. Figure 3C shows a statistical plot of the ratios of neurons with different transmitters. Nuclei were labeled with DAPI. Ruler: 25um.

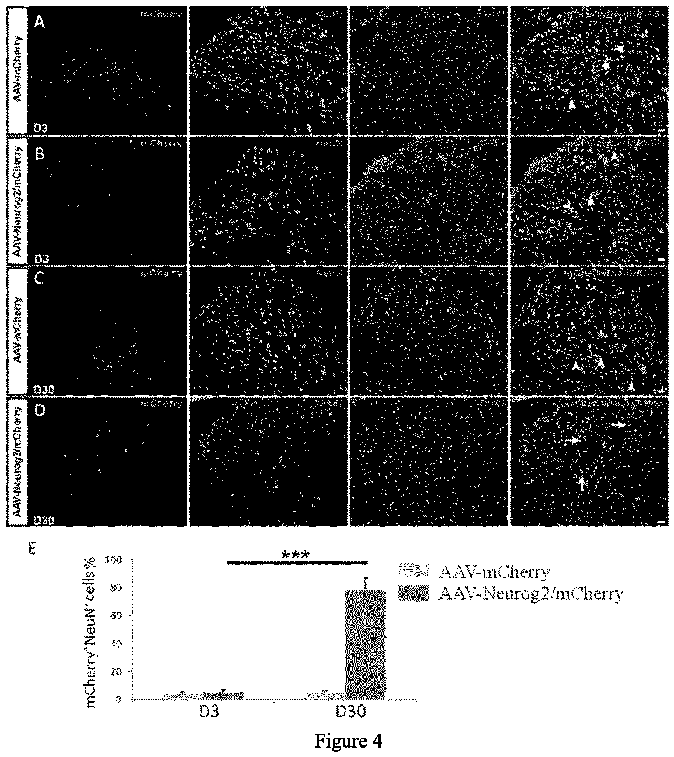

Figure 4 Neurog2 single factor can induce spinal cord astrocytes into neurons with high efficiency. Figure 4A, B show that 3 days after the control virus AAV-mCherry and virus AAV-Neurog2/mCherry were injected into the spinal cord of adult mice, immunoco-labeling showed that neither mCherry co-localized with NeuN. Figure 4C shows that 30 days after the control virus AAV-mCherry was injected into the spinal cord of adult mice, mCherry still did not co-localize with NeuN. Figure 4D shows that the vast majority of mCherry colocalized with NeuN 30 days after viral AAV-Neurog2/mCherry was injected into the adult mouse spinal cord. Figure 4E shows the statistics of neuron ratios at different times. Arrows and arrowheads represent mCherry⁺NeuN⁺ and mCherry⁺NeuN⁻ cells, respectively. "***" represents p<0.001. Ruler: 50um.

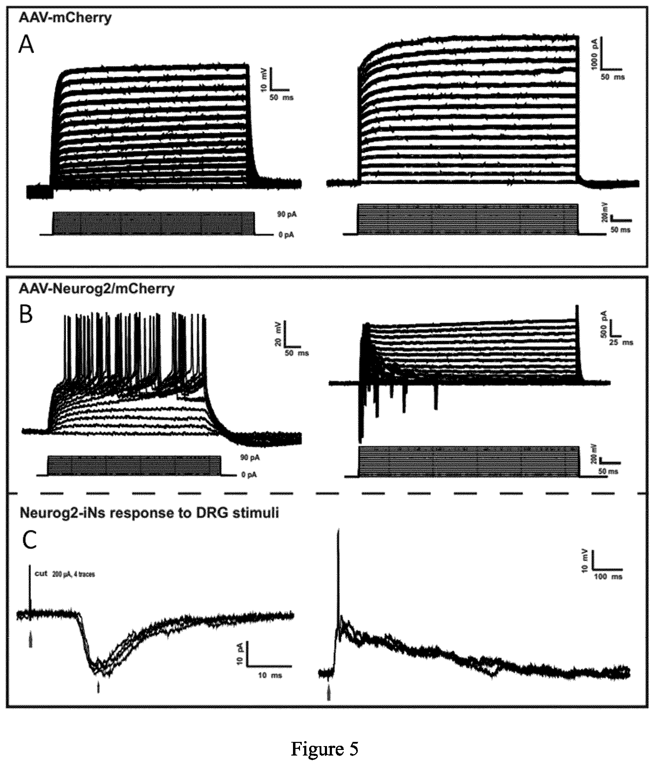

Figure 5 Neurog2-induced neurons in the spinal cord were confirmed to be active functional neurons by electrophysiological recording, and were able to respond to stimulation of the dorsal root ganglion (DRG). Figure 5A, B show electrophysiological recordings of spinal cord slices derived from mCherry⁺ cells infected with AAV-mCherry virus (A) and AAV-Neurog2/mCherry virus (B) for 30 days, in current-clamp or voltage-clamp mode, respectively, given gradients Voltage or current stimulation, resulting membrane voltage (left) and cell membrane current parameters (right). Figure 5C shows the electrophysiological response of mCherry⁺-induced neurons in the dorsal spinal cord after DRG stimulation. Green arrows represent the onset of stimulation.

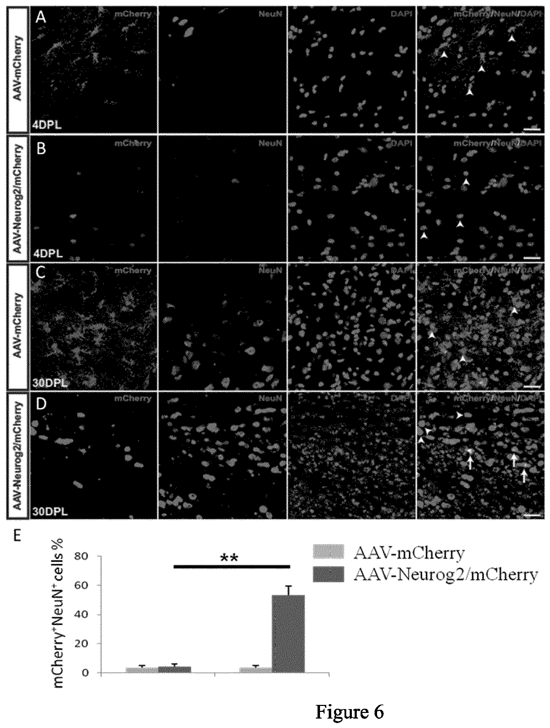

Figure 6 Neurog2 single factor can efficiently induce injured spinal cord astrocytes into neurons. Figure 6A, B show that 3 days after injection of the control virus AAV-mCherry and virus AAV-Neurog2/mCherry to injure the spinal cord of adult mice, immunoco-labeling showed that neither mCherry co-localized with NeuN. Figure 6C shows that mCherry still did not co-localize with NeuN 30 days after the control virus AAV-mCherry injection injured adult mouse spinal cord. Figure 6D shows that the vast majority of mCherry co-localized with NeuN 30 days after viral AAV-Neurog2/mCherry injection injured adult mouse spinal cord. Figure 6E shows a statistical graph of neuron ratios at different times. Arrows and arrowheads represent mCherry⁺NeuN⁺ and mCherry⁺NeuN⁻ cells, respectively. "**" represents p<0.01. Ruler: 50um.

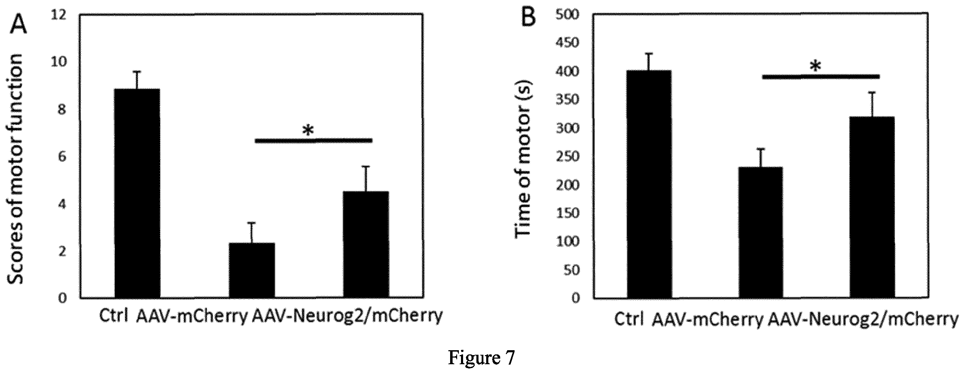

Figure 7 Neurog2 reprogrammed neurons can promote the recovery of motor function in mice with spinal cord injury. Figure 7A, B show the motor function of the control group (uninjured spinal cord) and the injured mice after 3 weeks of injection of virus AAV-mCherry and virus AAV-Neurog2/mCherry, respectively, Figure 7A scores the motor function of mice according to the BMS standard, Figure 7A, B 7B is the time the mice were in motion during the open field experiment. "*" represents p<0.05.

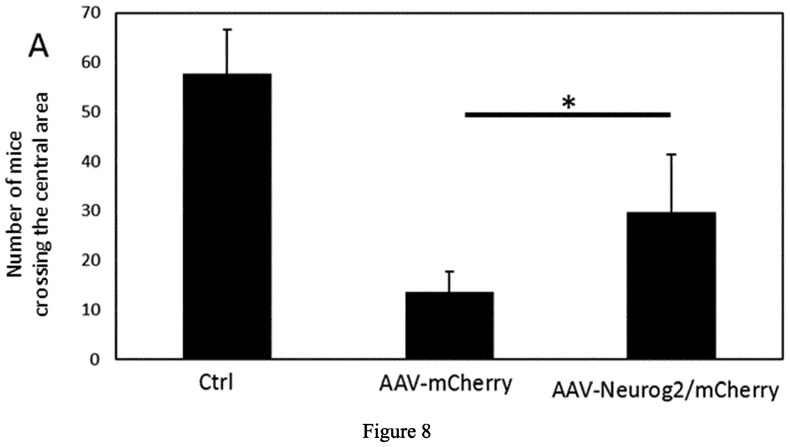

Figure 8 Neurog2 reprogramming neurons attenuates anxiety levels in mice with partial spinal cord injury. Figure 8A, B show the number of mice crossing the central area in the control group (uninjured spinal cord) and the injured mice 3 weeks after injection of virus AAV-mCherry and virus AAV-Neurog2/mCherry, respectively. "*" represents p<0.05.

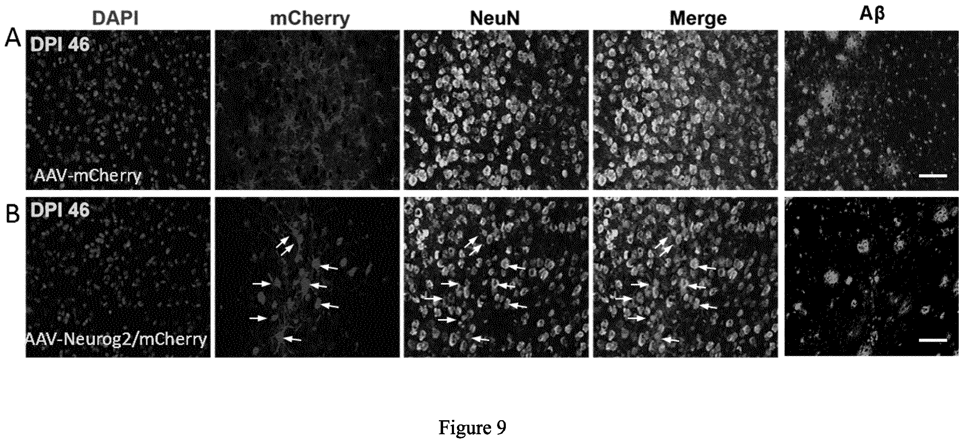

Figure 9 Neurog2 transdifferentiates astrocytes in AD mouse entorhinal cortex into neurons in vivo. Figure 9A, B show that 46 days after injection of the control virus AAV-mCherry and virus AAV-Neurog2/mCherry to damage the entorhinal cortex of 5XFAD mice, immunoco-labeling showed that neither mCherry in the control virus AAV-mCherry group co-localized with NeuN, nor did the group virus co-localize with NeuN. AAV-Neurog2/mCherry Most mCherry co-localized with NeuN, and the two groups of animal sections were simultaneously developed for Aβ coloration. Arrows represent mCherry⁺NeuN⁺ cells. Ruler: 50um.

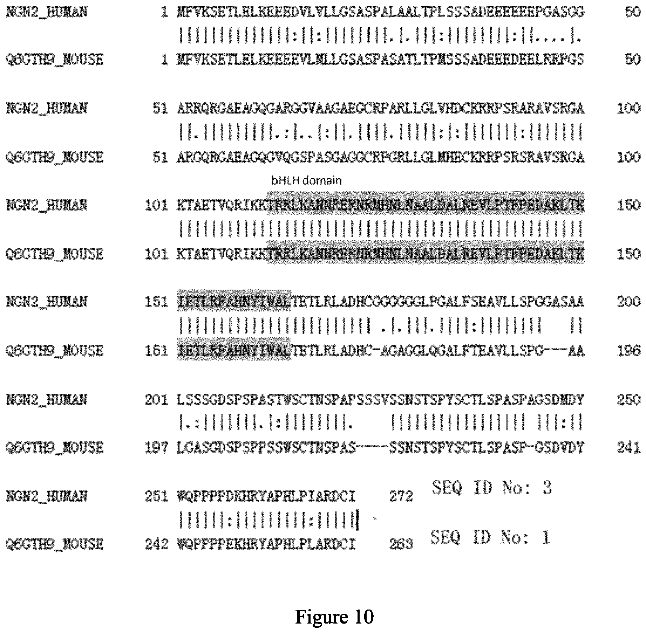

Figure 10 Alignment of amino acid sequences of mouse and human Neurog2.

### DETAILED DESCRIPTION

After extensive and in-depth research, the inventors found that regulating Neurog2 gene or its protein expression in glial cells can efficiently induce and transdifferentiate astrocytes into neurons with normal electrophysiological functions in vivo or in vitro, and unexpectedly found that not only astrocytes under normal conditions, but also astrocytes in a damaged environment can be transdifferentiated into functional neurons, which can play a role in neuroreparation. In particular, neurons derived from spinal cord-derived glial cells can respond to stimulation of the dorsal root ganglion (DRG) of the peripheral nervous system, showing that neurons induced by functional fragments of Neurog2 can function functionally integrated into spinal circuits and function. Therefore, the induction of functional fragments of Neurog2 is expected to be an effective method to stimulate the generation of new neuronal cells in adults, which can be widely used in the development of drugs for neurological diseases.

Compared with the prior art, the present invention mainly has the following competitive advantages:
(1) Compared with other discovered transdifferentiation factors, Neurog2 functional fragment can not only act on glial cells in normal environment, but also act on glial cells in damaged environment, breaking through glial cells/glial cells in damaged environment. In the stress state of nerve cells, neural connections with normal electrophysiological functions are obtained, resulting in the function of nerve repair.
(2) Compared with other discovered transdifferentiation factors, Neurog2 functional fragment can specifically and uniformly transdifferentiate glial cells into excitatory glutamatergic neurons or glutamatergic neuron populations, ensuring that in manipulation of drug development and application in neurological diseases.
(3) Compared with the transplantation of neuron clusters after induced neurons in vitro, in vivo AAV-mediated Neurog2-induced neurons is easier to operate, with higher neuron induction efficiency, and the induced neurons are more electrophysiologically mature and more efficient. Suitable for in vivo regenerative repair of neurological diseases.

### Neurog2 functional fragment or its promoter

Neurog2 (neurogenin 2) is a bHLH-like transcription factor, the main structural domains include DNA-binding domain, transcriptional regulatory domain (activation domain), oligomerization site and nuclear localization signal (nuclear localization), signal) and other functional areas. bHLH (basic Helix-Loop-Helix, basic helix-loop-helix) is a DNA-binding domain of Neurog2 that specifically regulates gene expression. The bHLH domain is well conserved in mammals including mice and humans.

The Neurog2 molecule from mouse has ID# 11924 in GenBank, and its protein sequence is shown in SEQ ID NO.:1; NCBI Reference Sequence:NM_009718.3; the CDS sequence is shown in SEQ ID NO.:2; the Neurog2 molecule from human has ID# 63973 in GenBank, and its protein sequence is shown in SEQ ID NO.:3., wherein the bHLH domain is located at positions 112 to 164; NCBI Reference Sequence:NM_024019.4; the CDS sequence is shown in SEQ ID NO.:4. The amino acid sequence alignment results of the mouse and human Neurog2 are shown in FIG. 10. The amino acid/protein sequences of mouse and human Neurog2 are shown below:
SEQ ID No: 1 (amino acid/protein sequence of mouse Neurog2) 
MFVKSETLEL KEEEEVLMLL GSASPASATL TPMSSSADEE EDEELRRPGS ARGQRGAEAE 60
QGVQGSPASG AGGCRPGRLL GLMHECKRRP SRSRAVSRGA KTAETVQRIK KTRRLKANNR 120
ERNRMHNLNA ALDALREVLP TFPEDAKLTK IETLRFAHNY IWALTETLRL ADHCAGAGGL 180
QGALFTEAVL LSPGAALGAS GDSPSPPSSW SCTNSPASSS NSTSPYSCTL SPASPGSDVD 240
YWQPPPPEKH RYAPHLPLAR DCI 263
SEQ ID No: 3 (amino acid/protein sequence of human Neurog2)

MFVKSETLEL KEEEDVLVLL GSASPALAAL TPLSSSADEE EEEEPGASGG ARRQRGAEAG 60
QGARGGVAAG AEGCRPARLL GLVHDCKRRP SRARAVSRGA KTAETVQRIK KTRRLKANNR 120
ERNRMHNLNA ALDALREVLP TFPEDAKLTK IETLRFAHNY IWALTETLRL ADHCGGGGGG 180
LPGALFSEAV LLSPGGASAA LSSSGDSPSP ASTWSCTNSP APSSSVSSNS TSPYSCTLSP 240
ASPAGSDMDY WQPPPPDKHR YAPHLPIARD CI 272

Neurog2 functional fragment refers to a Neurog2 sequence fragment with functions of regulating transcription and realizing re-differentiation, including but not limited to: full-length Neurog2 protein, and a sequence containing a conserved Neurog2bHLH domain and having more than 83% sequence homology with wild-type Neurog2 Fragment. Neurog2 functional fragments include mammalian polynucleotides encoding Neurogenin 2 transcription factors or their expressed protein fragments.

The functional fragment of Neurog2 can also be a variant of the Neurog2 protein sequence, in particular, the sequence homology between the variant of the Neurog2 protein functional fragment and SEQ ID NO.: 1 should not be less than 83%; or the protein functional sequence encoding Neurog2 and the sequence homology of SEQ ID NO.: 1 are not less than 90%; or the protein functional sequence encoding Neurog2 and the sequence homology of SEQ ID NO.: 1 are not less than 95%; or the protein functional sequence encoding Neurog2 and the sequence homology of SEQ ID NO.: 1 are not less than 98%; or the variant of Neurog2 protein functional fragment and the sequence homology of SEQ ID NO.:3 should not be less than 83%; or the protein functional sequence encoding Neurog2 and the sequence homology of SEQ ID NO.: 3 are not less than 90%; or the protein functional sequence encoding Neurog2 and the sequence homology of SEQ ID NO.: 3 are not less than 95%; or the sequence homology of the protein functional sequence encoding Neurog2 and SEQ ID NO.: 3 is not less than 98%;

The functional fragment of the Neurog2 can also be a variant of the Neurog2 polynucleotide sequence, in particular, the polynucleotide functional sequence encoding the Neurog2 and the sequence homology of SEQ ID NO.: 2 is not less than 80 %; or the sequence homology of the nucleic acid functional sequence encoding Neurog2 and SEQ ID NO.: 2 is not less than 90%; or the sequence homology of the nucleic acid functional sequence encoding Neurog2 and SEQ ID NO.: 2 is not less than 95%; or the sequence homology of the nucleic acid functional sequence encoding Neurog2 and SEQ ID NO.: 2 is not less than 98%; or the sequence homology of the polynucleic acid functional sequence encoding Neurog2 and SEQ ID NO.:4 is not less than 80%; or the sequence homology of the nucleic acid functional sequence encoding Neurog2 and SEQ ID NO.: 4 is not less than 90%; or the sequence homology of the nucleic acid functional sequence encoding Neurog2 and SEQ ID NO.: 4 is not less than 95%; or the sequence homology of the nucleic acid functional sequence encoding Neurog2 and SEQ ID NO.: 4 is not less than 98%;

The functional fragment of Neurog2 can also be obtained by activating the expression of Neurog2 gene by CRISPR/dCas9 targeting DNA, or by increasing the expression of Neurog2 by targeting RNA by CRISPR/Cas13;

The expression promoter of Neurog2 is not particularly limited, and can be any substance that promotes the expression and/or activity of the Neurog2 gene or its protein, such as small molecule compounds and stimulatory miRNAs. The person of ordinary skill in the art can screen Neurog2 promoters according to existing databases. It should be understood that, based on the transdifferentiation-inducing effect of Neurog2 on astrocytes disclosed in the present invention, the person of ordinary skill in the art can reasonably predict that any substance that promotes Neurog2 could induce transdifferentiation of astrocytes.

### Astrocytes

Astrocytes are the most abundant type of cells in the mammalian brain. They perform many functions, including biochemical support (such as forming the blood-brain barrier), providing nutrients to neurons, maintaining extracellular ion homeostasis, and participating in repair and scarring following brain and spinal cord injury. According to the content of glial filaments and the shape of cell processes, astrocytes can be divided into two types: the fibrous astrocytes are mostly distributed in the white matter of the brain and spinal cord, with slender processes, few branches, and a large number of glial filaments in the cytoplasm; protoplasmic astrocytes (protoplasmic astrocytes) are mostly distributed in the gray matter, with stubby cell processes and many branches.

The astrocytes that can be used in the present invention are not particularly limited, including various astrocytes derived from the central nervous system of mammals, such as those derived from the striatum, spinal cord, dorsal midbrain or cerebral cortex, preferably, derived from the dorsal midbrain or spinal cord.

In the present invention, astrocytes from various sources have higher inducing transformation efficiency. Typically, astrocytes are specific markers for GFAP, and astrocytes in gray matter have relatively low GFAP expression, but express either Acsbg1 or GS. And when induced by the methods of the present invention, these astrocytes exhibit markers specific to neuronal cells, such as NeuN.

### functional neuron

As used herein, the term "functional neuron" refers to neuronal cells with normal neuronal electrophysiological activity that are transdifferentiated from astrocytes in the presence of exogenous Neurog2 gene or protein. Typically, the functional neuronal cells have the following properties:
(a) NeuN, a marker for expressing neurons;
(b) capable of releasing action potentials and capable of forming synaptic connections.

In the present invention, the "functional neuron" is an excitatory neuron, especially a VGLUT2⁺ excitatory neuron, which is a glutamatergic neuron. Therefore, herein, the terms "excitatory neuron", "VGLUT2⁺ excitatory neuron", "glutamatergic neuron" are used interchangeably and all refer to the functional neurons of the present invention.

### Delivery system

The delivery system that can be used in the present invention is not particularly limited, and may be an expression vector containing a Neurog2 protein coding sequence capable of entering astrocytes. For example, a viral vector can be any viral vector that can utilize the characteristics of a virus to transmit its genome and bring genetic material into other cells for infection. Can occur in whole in vivo or in cell culture. Including lentivirus vector, adenovirus vector, adeno-associated virus vector, herpes virus vector, poxvirus vector.

The delivery system can also be a new type of nanoparticles for loading Neurog2 functional fragments and delivering them to target cells, such as liposome nanoparticles, metal nanoparticles, polymer nanoparticles and other delivery systems that can carry Neurog2 functional fragments.

The delivery system can also be exosomes coated with Neurog2 functional fragments, or modified red blood cells or bacteria coated with Neurog2 functional fragments.

In addition, the delivery system can also be combined with functionally targeted molecules, such as specific monoclonal antibodies targeting astrocytes, polypeptides, etc., which can better improve the target of Neuorog2 functional fragments on astrocytes. tropism and increased transdifferentiation efficiency.

### Induction method

The present invention also provides methods for inducing transdifferentiation of astrocytes into functional neuronal cells in vivo.

In vivo, a Neurog2-containing delivery system can be administered (eg, injected) to a desired subject where astrocyte-containing sites, such as the dorsal midbrain, cerebral cortex, or spinal cord, can target both intact and damaged nervous systems. Tissues are injected to induce transdifferentiation of astrocytes in specific parts of the nervous system.

In vitro, a Neurog2-containing delivery system can be administered (eg, injected) into NG2 glial cell clusters cultured in vitro, inducing the in vitro differentiation of functional neurons, and then transplant the functional neuron clusters cultured in vitro into the body by means of transplantation.

### Pharmaceutical composition and mode of administration

The present invention also provides a pharmaceutical composition which is a delivery system containing a Neurog2 functional fragment or a functional neuron mass after in vitro induction and transdifferentiation by the Neurog2 functional fragment.

The pharmaceutical composition of the present invention includes the above-mentioned expression vector (e.g., virus particle) of the present invention, or the exogenous Neurog2 protein itself, and a pharmaceutically acceptable carrier.

The pharmaceutical composition of the present invention usually contains 10 10-10 13 PFU/ml of AAV virus particles, preferably 10 11-10 13 PFU/ml of AAV virus particles, more preferably 10 10-10 12 PFU/ml of AAV virus particles AAV virus particles.

"Pharmaceutically acceptable carrier" refers to a carrier for administration of a therapeutic agent and includes various excipients and diluents. In general, the term refers to pharmaceutical carriers which are not themselves essential active ingredients and which are not unduly toxic after administration. Suitable carriers are well known to the person of ordinary skill in the art. Pharmaceutically acceptable carriers in the compositions may contain liquids such as water, saline, buffers. In addition, auxiliary substances such as fillers, lubricants, glidants, wetting or emulsifying agents, pH buffering substances and the like may also be present in these carriers. The carrier may also contain cell transfection reagents.

Usually, the pharmaceutical composition of the present invention can be obtained after mixing the expression vector and a pharmaceutically acceptable carrier.

The mode of administration of the composition of the present invention is not particularly limited, and representative examples include (but are not limited to): intravenous injection, subcutaneous injection, brain injection, intrathecal injection, spinal injection, and the like.

### Application

The Neurog2 functional fragment of the present invention can be used to prepare a drug for inducing astrocytes to generate functional neurons, so that the newly induced neurons can be used in various applications due to the reduction of the number of neurons, cell decline, apoptosis or neuron function decline related diseases. Wherein, the nervous system-related diseases include spinal cord injury, Alzheimer's disease (AD), Parkinson's disease (PD), neuronal death caused by stroke, and the like.

### Beneficial effects of the present invention

In the present invention, a single transcription factor Neurog2 can transdifferentiate astrocytes in the dorsal midbrain and spinal cord of adult mice into functional neurons. These induced neurons express neuronal hallmark molecules, can release action potentials, and can receive synaptic afferents from other neurons to establish synaptic connections. Therefore, this method is expected to be an effective method to stimulate the generation of new neuronal cells in adults, and thus be widely used in the treatment of neurological diseases, such as neurodegenerative diseases, traumatic diseases of the central nervous system, and so on.

The present invention will be further described below in conjunction with specific embodiments. It should be understood that these examples are only used to illustrate the present invention and not to limit the scope of the present invention. The experimental method of unreceipted specific conditions in the following examples, usually according to normal conditions, such as people such as Sambrook, molecular cloning: conditions described in laboratory manual (New York:Cold Spring Harbor Laboratory Press, 1989), or according to manufacture conditions recommended by the manufacturer.

### General approach

### NG2 cell culture

For preparative cultures of NG2 cells, cortical tissue from postnatal day 3-5 mice was removed and digested with 0.25% trypsin for 15 minutes. The blown cells were cultured in DMEM/F12 solution containing 10% serum for 7-9 days. The flasks were then shaken and the supernatant collected, centrifuged to resuspend the cells and seeded on poly-D-lysine (Sigma) coated coverslips at 37°C in a 5% CO2 incubator with N2 supplement (STEMCELL^{™}), 10 ng/ml platelet-derived growth factor (PDGF, Invitrogen), 10 ng/mL epidermal growth factor (EGF, Invitrogen), and 10 ng/mL fibroblast growth factor 2 (FGF2, Invitrogen) in serum-free DMEM medium (Gibco) for 3 days.

### Immunochromic

The immunochromatization of cultured cells refers to "Direct conversion of fibroblasts to functional neurons by defined factors" (Vierbuchen, T. et al. Nature 463, 1035-1041 (2010)). Immunochromatization of tissue sections combined with in situ hybridization and immunoassay Double-labeling experiments for color development were performed according to published methods. The primary antibodies used for immunochromatography include: mouse anti-GFAP (Millipore, 1:1,000), mouse anti-NeuN (Millipore, 1:100), anti-Dsred (Clontech, 1:500), mouse anti-Dsred (Santa Cruz, 1:100), rabbit anti-Acsbg1 (Abcam, 1:100), rabbit anti-NG2 (Millipore, 1:200), rabbit anti-Iba1 (Wako, 1:500), mouse anti-CNPase (Abcam), 1:500), mouse anti-O4 (Millipore, 1:500),. FITC-, Cy3- and Cy5-conjugated secondary antibodies were purchased from Jackson Immunoresearch.

### Stereotactic injection of AAV virus

AAV virus was performed with reference to mouse brain atlas. After virus injection, the dorsal midbrain and spinal cord were collected at different time points for immunochromatization or brain slice recording. The concentration of virus injection in the intact spinal cord and the injured spinal cord, the speed and the injection volume of each injection are consistent with the brain region, and the injection in the spinal cord is at an angle of 30°.

### Transplantation of induced neurons

The mice used for transplantation were seven-week-old NOD-scid mice. The cells were digested with Accutase for 4-6 days, and the supernatant was removed by centrifugation so that the density of the cells was about 2×10 5 cells/µl after concentration, and 2 µl of each mouse brain was transplanted, that is, a total of 4×10 5 cells. Histochemical and electrophysiological tests were performed 2-4 weeks after transplantation.

### Spinal cord injury detection model

The loss of sensory afferents after thoracic spinal cord injury promotes the weakening of the descending inhibitory system of the brainstem and leads to hypersensitivity of the tail to external stimuli. We used the tail-flick experimental model to measure the sensory ability of mice by measuring the response delay time of the tails of two groups of mice under thermal stimulation at 48°C and 52°C. Motor function of mice was scored according to BMS criteria. The test method refers to Basso Mouse Scale for locomotion detects differences in recovery after spinal cord injury in five common mouse strains. J Neurotrauma, 2006.23(5):p.635-59.

### Open field experiment

were used to assess the level of anxiety in animals. It is generally believed that the activity of mice in the central area of the open field is related to the degree of anxiety. Normal mice will frequently shuttle through the central area of the open field (Zone Crossing), while anxious mice tend to move more around the square (Peripheral). The experimental method refers to the open field as a paradigm to measure the effects of drugs on anxiety-like behaviors: a review. European Journal of Pharmacology, 2003. 463(1-3): p.3-33.

### Embodiments:

### Embodiment 1. Neurog2 transdifferentiates NG2 glial cells into functional neurons in vitro

### (1) Plasmid construction and virus infection

On a vector template of FUGW-IRES-EGFP (for vector information ref. Efficient transfer, integration, and sustained long-term expression of the transgene in adult rat brains injected with a lentiviral vector. Proc Natl Acad Sci U S A 93:11382-11388) The human NG2 promoter (SEQ ID No.: 5) was cloned to replace the CAG promoter, and the CDS (SEQ ID No.: 4) fragment derived from the human Neurog2 gene was constructed into this lentiviral vector to generate hNG2-hNeurog2- IRES-EGFP lentiviral plasmid. For packaging of lentiviral vectors, refer to "Production and purification of lentiviral vectors" (Tiscomia, G., Singer, O. & Verma, I.M. Nat. Protoc. 1, 241-245 (2006)).

NG2 cells were plated for 24 hours before lentivirus was added, and the medium was changed 24 hours after infection: DMEM/F12, B27, Glutamax and penicillin/streptomycin. After infection 6-7, brain-derived neurotrophic factor (BDNF; PeproTech, 20 ng/ml) was added to the medium every three days.

### (2) Neurog transdifferentiates NG2 cells into neurons

Most of the cultured mouse NG2 cells were immunopositive for the NG2 glial cell marker NG2, and a small number of cells expressed oligodendrocyte marker molecules O4 and CNPase, the expression of neuron marker molecule Tuj 1 and stem cell marker molecules Sox2 and Oct4 was not detected.

10 days after NG2 cells were transfected with hNG2-Neurog2-IRES-GFP lentivirus, most NG2 cells showed typical neuron morphology and expressed neuron marker molecule Tuj1. 21 days after lentivirus infection, the induced cells also expressed mature neuron marker molecules NeuN and MAP2. Electrophysiological recordings showed that induced neurons could generate action potentials, and the vast majority of induced neurons could record spontaneous postsynaptic currents, suggesting that these neurons could form functional synapses.

### (3) Neurons induced by NG2 cells can survive transplantation in vivo

Whether the transdifferentiated neurons induced in vitro can survive and function in vivo is the key to whether they can be used for disease treatment. Neurog2 immunohistochemical experiments were performed two weeks after NG2 cells-induced neurons were transplanted into the cerebral cortex. We found that the transplanted cells could attach to the edge of the cortex, and some cells could extend neurites deeper into the cortex. The results of immunofluorescence colocalization experiments showed that some of the transplanted cells expressed the neuron marker molecule Tuj1, indicating that the induced neurons could survive and express neuronspecific marker molecules.

### Embodiment 2. Neurog2 transdifferentiates adult dorsal midbrain astrocytes into neurons in vivo

### (1) Adeno-associated virus plasmid construction and virus infection

The GFAP promoter (SEQ ID No.: 6) was cloned on the vector template of AAV-FLEX-Arch-GFP (Addgene, #22222) to replace the CAG promoter and retain the CMV enhancer, and then replace the GFP with the coding frame of mCherry. AAV-mCherry plasmid (control). The CDS (SEQ ID No.: 2, NCBI: NM_009718.3) derived from the mouse Neurog2 gene was cloned into the AAV-mCherry plasmid to obtain the AAV-mNeurog2/mCherry plasmid. The target gene can be specifically targeted under the action of the GFAP promoter astrocytes.

### (2) Neurog2 transdifferentiates astrocytes into neurons

The virus AAV-mCherry or AAV-mNeurog2/mCherry was injected into the lateral tectum of adult wild-type mice, and then brain tissue samples were collected at different time points. Co-immunolabeling showed that mCherry did not co-localize with NeuN either in mice injected with the control virus AAV-mCherry (Fig. 1A) or with the virus AAV-Neurog2/mCherry (Fig. 1B) 3 days after virus injection. In mice injected with the control virus AAV-mCherry 30 days after virus injection, co-immunolabeling showed that mCherry still did not co-localize with NeuN. However, in mice injected with the virus AAV-Neurog2/mCherry, the vast majority of mCherry colocalized with NeuN (88.2±6.3%). This indicates that Neurog2 successfully induces astrocytes into neurons.

### (3) Induced neurons are functional neurons

To demonstrate that induced neurons are functionally active neurons, we performed electrophysiological recordings. We found that mCherry⁺ cells sliced 30 days after midbrain infection with AAV-mCherry virus did not generate action potentials (Fig. 2A), suggesting that targeting astrocytes in vivo by AAV-mCherry virus did not alter their electrophysiological properties. When recording Neurog2/mCherry virus-infected brain slices, we found that 19 of the 20 recorded cells exhibited inward Na⁺ currents and outward K⁺ currents in voltage-clamp mode (19/20). Furthermore, 19 recorded cells were able to fire multiple action potentials (19/20). Importantly, 17 of the recorded cells were able to generate excitatory postsynaptic currents (EPSCs) (17/20) (Fig. 2B), which disappeared after the addition of the blocker NBQX and washed out Postsynaptic current signaling reappears. This indicates that Neurog2-induced neurons are integrated into neural circuits to establish synaptic connections and are functional neurons.

### (4) The induced neurons are all excitatory neurons

We characterized Neurog2-induced neurotransmitter properties. VGLUT2/Gad1 mRNA and mCherry protein were stained by in situ hybridization and immunohistochemistry. The experimental results showed that 30 days after infection, AAV-Neurog2/mCherry virus could reprogram midbrain astrocytes into VGLUT2⁺ excitatory neurons (Fig. 3A, C), but not Gad1⁺ inhibitory neurons (Fig. 3B, C). Thus, we demonstrate that Neurog2 single factor can reprogram midbrain astrocytes into glutamatergic neurons.

### Embodiment 3 Neurog2 transdifferentiates adult mouse normal spinal cord astrocytes into neurons in vivo

### (1) Plasmid construction and virus infection

Using the adeno-associated virus vector AAV-mCherry plasmid described in Embodiment 2, the CDS (SEQ ID No.: 4, NCBI: NM _024019.4) derived from the human Neurog2 gene was cloned into AAV-mCherry to obtain AAV-hNeurog2/mCherry plasmid.

### (2) Neurog2 transdifferentiates astrocytes into neurons

We injected AAV-mCherry virus and AAV-hNeurog2/mCherry virus into the dorsal spinal cord of adult mouse thoracic segment (Thoracic, T8-T10) 3 days later and found that both viruses can specifically infect astrocytes. Co-immunolabeling showed that mCherry did not co-localize with NeuN either in mice injected with the control virus AAV-mCherry (Fig. 4A) or with the virus AAV-Neurog2/mCherry (Fig. 4B) 3 days after virus injection. In mice injected with the control virus, AAV-mCherry, 30 days after virus injection, co-immunolabeling showed that mCherry still did not co-localize with NeuN (Fig. 4C). However, in mice injected with the virus AAV-Neurog2/mCherry, the vast majority of mCherry colocalized with NeuN (Fig. 4D). This indicated that Neurog2 successfully induced spinal astrocytes into neurons.

### (3) Induced neurons are functional neurons

We analyzed the electrophysiological properties of Neurog2 reprogrammed neurons in the spinal cord. Through whole-cell recordings in brain slices, it was found that 30 days after AAV-mCherry virus infection, mCherry⁺ cells could not generate action potentials when stimulated with intracellular gradient currents, nor could they generate inward currents when stimulated in voltage-clamp mode (Fig. 5A). These features are consistent with those reported for astrocytes. On the other hand, after 30 days of AAV-Neurog2/mCherry virus infection, most mCherry ⁺ cells were able to fire multiple action potentials under gradient current stimulation, and were able to generate inward Na ⁺ current and outward K ⁺ current under voltage-clamp mode stimulation (Fig. 5B). We further examined whether Neurog2 reprogrammed neurons integrate into existing neural circuits. We removed the spinal cord along with the DRG section for electrophysiological testing. When we stimulated the DRG, we were able to detect excitatory postsynaptic currents (EPSC) and excitatory postsynaptic potential (EPSP) signals from neurons induced in the dorsal spinal cord. (Fig. 5C), indicating that Neurog2 not only reprograms neurons, but that reprogrammed neurons in the intact spinal cord can integrate into neural circuits and exert potential functions.

### Embodiment 4 Neurog2 transdifferentiates adult mouse spinal cord astrocytes into neurons in vivo

Spinal cord injury (SCI) is a kind of central nervous system damage disease, which is accompanied by the death of spinal cord neurons and the formation of glial scars. In vivo neuronal reprogramming to convert astrocytes into neurons may alleviate the damage caused by SCI and is expected to become a new treatment method.

### (1) Neurog2 transdifferentiates astrocytes into neurons

We attempted to use Neurog2 to reprogram glial cells into neurons in the context of injury. First, a mouse T8-T10 spinal cord amputation model was constructed (refer to the method of McDonough A, Monterrubio A, Ariza J, et al.Calibrated Forceps Model of Spinal Cord Compression Injury.Jove-Journal of Visualized Experiments 2015.), after injury AAV-mCherry virus and AAV-mNeurog2/mCherry (Example 2) or AAV-hNeurog2/mCherry (Example 3) were injected into both sides of the injured spinal cord immediately. Co-immunolabeling showed that mCherry did not co-localize with NeuN either in mice injected with the control virus AAV-mCherry (Fig. 6A) or with the virus AAV-mNeurog2/mCherry (Fig. 6B) 3 days after virus injection. In mice injected with the control virus, AAV-mCherry, 30 days after virus injection, co-immunolabeling showed that mCherry still did not co-localize with NeuN (Fig. 6C). However, in mice injected with the virus AAV-mNeurog2/mCherry, some mCherry could be found to co-localize with NeuN (Fig. 6D). This indicated that Neurog2 successfully induced the injured spinal cord astrocytes into neurons. Similarly, the adeno-associated virus vector plasmid AAVhNeurog2/mCherry loaded with human-derived Neurog2 can also successfully induce injured spinal cord astrocytes into neurons.

### (2) Repair of spinal cord injury model

Neurog2 reprogrammed neurons after spinal cord injury have electrophysiological characteristics and can accept external signal input. Therefore, we used a variety of animal models to evaluate the neuronal repair ability of mice with spinal cord injury after neuronal induction. Using a spinal cord injury detection model, we found that Neurog2 reprogrammed neurons greatly contributed to the recovery of sensory and motor functions in mice with spinal cord injury.

We also used the spinal cord T8-T10 transection model, and injected AAV-mCherry virus and AAV-Neurog2/mCherry virus into both sides of the injured spinal cord, which were used as the experimental group and the control group, and 6 male untreated mice of the same age were selected as the Reference Group (Ctrl).

We tested mouse motor function three weeks after virus injection, which was scored according to BMS criteria (Basso, D.M., et al., J Neurotrauma, 2006.). The test data showed that the mice in the Ctrl group were basically able to maintain the exercise capacity of 9 points, while the exercise ability of the mice in the AAV-mCherry virus group was 2.3 points, and the mice injected with AAV-Neurog2/mCherry increased to 4.5 points (Figure 7A). Then, the exercise ability of the mice was further evaluated by the open field test. Through analysis, it was found that the two groups of mice injected with the virus had less exercise time within 15 minutes than the Ctrl group, but the exercise time between the two groups of virus-injected mice was significantly different. Statistical difference (Figure 7B). This indicates that Neurog2 reprogramming neurons can promote the recovery of motor function in mice with spinal cord injury.

We also used the mouse square assay to assess the level of anxiety in mice after injury to test whether Neurog2-reprogrammed neurons can reduce anxiety levels in mice with partial spinal cord injury, acute/chronic pain due to spinal cord injury, especially Chronic pain often leads to anxiety. In mouse models of spinal cord injury, which are usually accompanied by varying degrees of anxiety, we found that neurog2 reprogrammed neurons also achieved great relief of anxiety in spinal cord injury mice. In open field experiments, it is generally believed that the activity of mice in the central area of the open field is related to the degree of anxiety. Normal mice frequently shuttle through the central area of the open field, while anxious mice tend to move more around the open field.

We also analyzed the performance of the three groups of experimental mice. The results showed that at 3 weeks, the mice in the Ctrl group frequently shuttled through the central area of the open field, while the mice in the AAV-mCherry group tended to move more around the open field. AAV-Neurog2/ The mice in the mCherry group shuttled more frequently than the mice in the AAV-mCherry group (Fig. 8). These results suggest that Neurog2 reprogramming neurons can reduce anxiety levels in some mice with spinal cord injury.

### Embodiment 5 Neurog2 transdifferentiates astrocytes in AD mouse entorhinal cortex into neurons in vivo

We injected AAV-mCherry virus as well as AAV-hNeurog2/mCherry virus into the entorhinal cortex of 5xFAD of 6-month AD model mice. In mice with mCherry, immunoco-labeling showed that mCherry did not co-localize with NeuN. At 46 days after virus injection, in mice injected with the control virus AAV-mCherry, co-immunolabeling showed that mCherry still did not co-localize with NeuN (Figure 9A). However, in mice injected with the virus AAV-Neurog2/mCherry, the vast majority of mCherry co-localized with NeuN (Fig. 9B), while the expression range of Aβ was reduced.

These results demonstrate that Neurog2 successfully transdifferentiates astrocytes in the entorhinal cortex of AD mice into neurons in vivo and reduces Aβ expression. Furthermore, these results suggest that the transdifferentiated transcription factor Neurog2 provides new technologies and drugs for the treatment of Alzheimer's disease.

The preferred embodiments of the present invention have been specifically described above, but the present invention is not limited to the described embodiments, and those skilled in the art can also make various Equivalent modifications or substitutions are included within the scope defined by the claims of the present application.

## Claims

1. A Neurog2 functional fragment for use in (i) a preparation of a pharmaceutical composition for nerve induced a glial cell to form a functional neuron; and/or (ii) a preparation of a pharmaceutical composition for a nervous system disease.

2. The Neurog2 functional fragment for use according to claim 1, wherein the glial cell is selected from human or non-human mammalian astrocyte, NG2 glial cell, oligodendrocyte or microglia cell; preferably, the glial cell is astrocyte.

3. The Neurog2 functional fragment for use according to claim 2, wherein the glial cell is derived from spinal cord, dorsal midbrain or cerebral cortex; preferably, the glial cell is derived from spinal cord and dorsal midbrain; wherein, the glial cell is glial cell in a normal state or a damaged state.

4. The Neurog2 functional fragment for use according to claim 1, wherein the Neurog2 functional fragment is a functional Neurog2 protein or a nucleotide sequence encoding Neurog2: wherein a sequence homology of the Neurog2 protein sequence and SEQ ID NO.: 1 or SEQ ID NO.: 3 is not less than 83%; more preferably, the sequence homology of the Neurog2 protein sequence and SEQ ID NO.: 1 is not less than 90%; preferably, the sequence homology of Neurog2 protein and SEQ ID NO.: 1 is not less than 95%; wherein the nucleic acid sequence encoding Neurog2 has no less than 80% homology with SEQ ID NO.: 2 or SEQ ID NO.: 4.

5. The Neurog2 functional fragment for use according to claim 1, wherein the functional neuron comprises a glutamate neuron or a group of glutamate neurons.

6. A Neurog2 functional fragment delivery system, wherein the Neurog2 functional fragment delivery system can be used in vivo or in vitro to induce a glial cell to differentiate into a functional neuron; the glial cell is in normal state or damaged state.

7. The Neurog2 functional fragment delivery system according to claim 6, wherein the Neurog2 functional fragment delivery system is an expression vector containing a Neurog2 functional fragment, wherein the expression vector can enter the glial cell and express exogenous Neurog2 protein in an astrocyte.

8. The Neurog2 functional fragment delivery system according to claim 6 or 7, wherein the Neurog2 functional fragment delivery system is a viral vector, the viral vector is selected from the group consisting of adeno-associated viral vector, adenoviral vector, retroviral vector and lentiviral vector.

9. The Neurog2 functional fragment delivery system according to claim 8, wherein the viral vector comprises a glial cell specific promoter and the Neurog2 functional fragment.

10. The Neurog2 functional fragment delivery system according to claim 9, wherein the viral vector promoter is selected from the group consisting of GFAP promoter, NG2 promoter, Aldh1L1 promoter, IBA1 promoter, CNP promoter, LCN2 promoter and genetically engineered promoter variants.

11. The Neurog2 functional fragment delivery system according to claim 8-10, wherein the viral vector is a GFAP-AAV vector or a NG2-lentiviral vector.

12. A host cell containing an exogenous Neurog2 functional fragment, wherein the host cell is derived from a glial cell cultured in vitro or a glial cell in a normal state or in a damaged state, the host cell transdifferentiate to form a functional neuron or a group of functional neuronal cells.

13. The host cell according to claim 12, wherein the functional neuron has the following characteristics: (a) expressing a neuron marker NeuN; (b) capable of releasing action potentials and capable of forming synaptic connections.

14. The host cell according to claim 12, wherein the group of functional neuronal cells have the following characteristics: (a) at least 50% of the neurons, preferably at least 60%, 70%, 80%, 90%, or 100% of the neurons express the neuron marker NeuN; (b) capable of releasing action potentials and capable of forming synaptic connections.

15. An application of the Neurog2 functional fragment delivery system according to claim 6-11 or the host cell according to claim 12-14, wherein the Neurog2 functional fragment delivery system or the host cell can be used to prepare a pharmaceutical composition for a nervous system injury or a nerve repair, the pharmaceutical composition further comprises a pharmaceutically acceptable carrier.

16. The application according to claim 15, wherein the nervous system injury comprises spinal cord injury, epilepsy, Alzheimer's disease (AD), Parkinson's disease (PD), neuronal death caused by stroke or irreversible loss.

17. A pharmaceutical composition comprising (A) a Neurog2 functional fragment; and/or (B) a delivery system as claimed in claim, or (C) a host cell as claimed in claim and (D) a pharmaceutically acceptable excipient.
